# EUROPEAN PATENT APPLICATION

(11) **EP 4 748 829 A1**
(43) Date of publication of application: **27.05.2026**
(21) Application number: 24842049.9
(22) Date of filing: 21.05.2024
(51) Int. Cl.: C07C 211/55, C08K 5/18, C08L 7/00

(54) **ANTIOXIDANT RESISTANT TO DAMP-HEAT AGING AND OZONE AGING, AND RUBBER COMPOSITION AND USE THEREOF**

(30) Priority: 18.07.2023 CN 202310883162
(71) Applicant: Sennics Co., Ltd., Shanghai, 200120 (CN)
(72) Inventor: GAO, Yang, Shanghai 200120 (CN); ZHANG, Jin, Shanghai 200120 (CN); GUO, Xiangyun, Shanghai 200120 (CN)
(74) Representative: V.O.
(86) International application number: PCT/CN2024/094389
(87) International publication number: WO 2025/016049

(57) **Abstract**

Provided in the present invention are a compound of formula A, which can be used as an antioxidant, an antioxidant composition comprising the compound of formula A and an antioxidant STMQ, a rubber composition comprising the antioxidant composition or the compound of formula A, and the use of the rubber composition. The compound of formula A is as described in the specification. The antioxidant and the antioxidant composition of the present invention can significantly improve the damp-heat aging resistance of a tire rubber material, and also have good ozone aging resistance, thereby prolonging the service life of a tire in special and harsh environments.

## Description

### TECHNICAL FIELD

The present invention belongs to the field of rubber materials and relates to an antidegradant resistant to damp-heat aging and ozone aging, and a rubber composition and use thereof.

### BACKGROUND

During the storage, processing and use of rubber products, intrinsic factors and environmental factors (heat, oxygen, ozone, ultraviolet radiation, chemicals, etc.) cause a decline or loss in the performance of such products. Oxygen is an important factor in rubber aging, while the absorption of thermal energy is the main factor for the pyrolysis of polymeric materials in vacuum or inert environments. Meanwhile, thermal energy can also accelerate the oxidation reaction of rubber products. Ozone is more reactive than oxygen, so its attack on rubber, especially unsaturated rubbers, is more severe than that of oxygen. The reaction between ozone and the double bonds in rubber will accelerate the formation and propagation of cracks; in addition, an increase in a concentration of ozone correspondingly shortens the time for cracks to appear and increases a growth rate of the crack.

At present, in tire formulations, it is common to simultaneously add physical antidegradants and chemical antidegradants to improve the ozone aging resistance of tires, including static and dynamic performance. Among them, chemical antidegradants, especially p-phenylenediamine type antidegradants, such as 6PPD (N-1,3-dimethylbutyl-N'-phenyl-p-phenylenediamine), IPPD (N-isopropyl-N'-phenyl-p-phenylenediamine), etc., are widely used in the tire industry due to their good comprehensive protective performance (ozone, thermo-oxidative, and dynamic fatigue resistance).

Water (precipitation, humidity, condensation, etc.) also plays an important role in accelerating the aging of rubber products. The extraction effect of water often accelerates the loss of antidegradants on the surface of rubber products, thereby accelerating aging and leading to a decline in physical and mechanical performance.

When the external environment for the use and storage of rubber products becomes more severe, such as in a high-temperature and high-humidity environment, there are significant differences in the water solubility (water extraction resistance) of different antidegradants, leading to a noticeable decrease in the concentration/proportion of antidegradants in the rubber composition and ultimately a significant reduction in the protective effect of rubber products. Relevant literature discloses that both antidegradants IPPD and 6PPD have poor hot water extraction resistance.

### SUMMARY OF THE INVENTION

Aiming at the problems existing in the prior art, the present invention provides an antidegradant with damp-heat aging resistance and ozone aging resistance, an antidegradant composition comprising the antidegradant, a rubber composition comprising the antidegradant or the antidegradant composition, and uses thereof. The antidegradant and antidegradant composition of the present invention can, on the premise of maintaining the original physical properties and thermo-oxidative aging resistance of the rubber composition (e.g., tire rubber composition) unchanged, significantly improve the damp-heat aging resistance of the rubber composition while also having good ozone aging resistance, thereby prolonging the service life of rubber products (e.g., tires) under special and harsh environments.

Specifically, one aspect of the present invention provides a compound of formula A that can be used as an antidegradant: 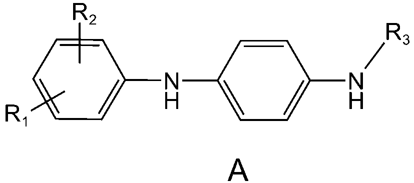 in formula A, R₁, R₂ and R₃ are each independently selected from C1-C8 alkyl.

In one or more embodiments, in formula A, R₁ and R₂ are each independently selected from C1-C2 alkyl, and R₃ is selected from C3-C6 alkyl, preferably selected from C4-C6 alkyl.

In one or more embodiments, a compound of formula A is a compound of formula I, a compound of formula II, a compound of formula IV, or a compound of formula VI:

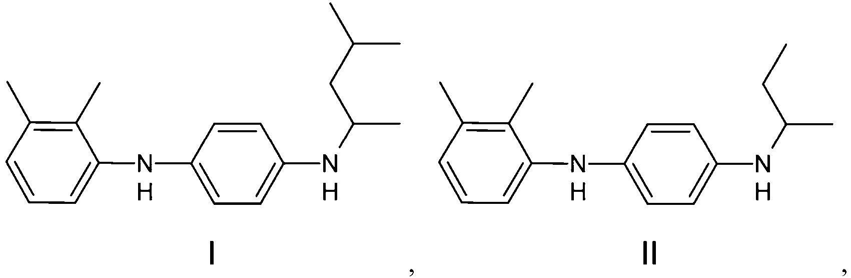

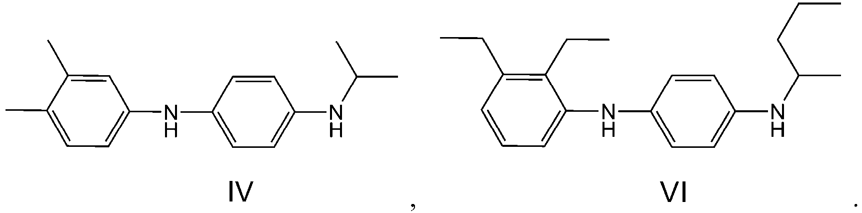

In one or more embodiments, a compound of formula A excludes a compound that R₁ and R₂ are methyl and R₃ is an isopropyl.

Another aspect of the present invention provides an antidegradant composition, wherein the antidegradant composition comprises antidegradant STMQ and a compound of formula A according to any embodiment herein, the antidegradant STMQ is a polymer of 2,2,4-trimethyl-1,2-dihydroquinoline wherein a total mass fraction of dimers, trimers, and tetramers of 2,2,4-trimethyl-1,2-dihydroquinoline is ≥ 80%.

In one or more embodiments, in the antidegradant composition, a mass ratio of the compound of formula A to the antidegradant STMQ is 1:1 to 3:1, preferably 1.5:1 to 2.5:1.

Another aspect of the present invention provides a rubber composition, wherein the raw materials of the rubber composition comprise 100 parts by mass of a diene elastomer and 1 to 5 parts by mass of a compound of formula A according to any embodiment herein, or comprise 100 parts by mass of a diene elastomer and 1 to 5 parts by mass of an antidegradant composition according to any embodiment herein.

In one or more embodiments, the diene elastomer comprises natural rubber and butadiene rubber; preferably, a mass ratio of the natural rubber to the butadiene rubber is 1:2 to 2:1.

In one or more embodiments, the raw materials of the rubber composition further comprise 30 to 70 parts by mass of a reinforcing filler; preferably, the reinforcing filler is carbon black.

In one or more embodiments, the raw materials of the rubber composition further comprise 1 to 10 parts by mass of an activator; preferably, the activator is zinc oxide.

In one or more embodiments, the raw materials of the rubber composition further comprise 2 to 15 parts by mass of a softener; preferably, the softener comprises aromatic oil and stearic acid in a mass ratio of 1:1 to 5:1.

In one or more embodiments, the raw materials of the rubber composition further comprise 0.5 to 3 parts by mass of sulfur.

In one or more embodiments, the raw materials of the rubber composition further comprise 0.2 to 2 parts by mass of an accelerator, and the accelerator is preferably N-tert-butylbenzothiazole-2-sulphenamide.

Another aspect of the present invention provides a rubber product, wherein the rubber product comprises a rubber composition according to any embodiment herein; preferably, the rubber product is a tire.

The present invention further provides a use of a compound of formula A according to any embodiment herein or an antidegradant composition according to any embodiment herein in improving the damp-heat aging resistance and/or ozone aging resistance of a rubber composition or a rubber product.

### DETAILED DESCRIPTION OF THE INVENTION

To enable those skilled in the art to understand the features and effects of the present invention, the following is a general description and definition of terms and words mentioned in the specification and claims. Unless otherwise specified, all technical and scientific terms used herein are intended to be the ordinary meaning of the knowledge of the present invention by those skilled in the art, and in case of a conflict, the definition of this specification shall prevail.

The theories or mechanisms described and disclosed herein, whether right or wrong, should not limit the scope of the present invention in any way, i.e., the content of the present invention may be practiced without limitation to any particular theory or mechanism.

Herein, the terms "comprising", "including", "containing" and the like encompass terms "consisting essentially of" and "consisting of". For example, where it is disclosed herein that "A comprises B and C", "A consists essentially of B and C" and "A consists of B and C" should be considered to be disclosed herein.

Herein, all features, such as numerical values, quantities, amounts and concentrations, which are defined by numerical ranges or percentage ranges, are only for the sake of simplicity and convenience. Accordingly, the recitation of numerical ranges or percentage ranges shall be construed as covering and specifically disclosing all possible sub-ranges and individual values (including integers and fractions) in the range.

Herein, unless otherwise specified, a percentage refers to a mass percentage, and a ratio refers to a mass ratio.

Herein, when embodiments or Examples are described, it should be understood that they are not intended to limit the disclosure to these embodiments or Examples. On the contrary, all alternatives, improvements and equivalents of the methods and materials described in the present disclosure can be covered within the scope defined by the claims.

Herein, for the sake of brevity of description, all possible combinations of various technical features in the various embodiments or Examples are not described. Therefore, as long as there is no contradiction in the combination of these technical features, the various technical features in the various embodiments or Examples can be combined in any combination, and all possible combinations should be considered to be within the scope of this specification.

In the present invention, the compound of formula A has the following structure: 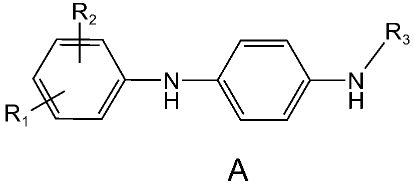 in formula A, R₁, R₂ and R₃ are each independently selected from C1-C8 alkyl.

In the present invention, "alkyl" refers to a linear or branched monovalent saturated hydrocarbon group. The alkyl group may contain 1 to 8 carbon atoms (C1-C8 alkyl). Examples of the alkyl group include, but are not limited to, methyl, ethyl, propyl, isopropyl, 1-methylpropyl, isobutyl, 1-methylbutyl, and 1,3-dimethylbutyl.

In formula A, R₁ may be located at the ortho-, meta-, or para-position to the -NH- group on the benzene ring, and R₂ may be located at the ortho-, meta-, or para-position to R₁. In some embodiments, R₁ is located at the ortho-position to the -NH- group on the benzene ring, and R₂ is located at the ortho-position to R₁. In some embodiments, R₁ is located at the meta-position to the -NH- group on the benzene ring, and R₂ is located at the para-position to the -NH- group on the benzene ring.

Preferably, in formula A, R₁ and R₂ are each independently selected from C1-C4 alkyl, more preferably selected from C1-C3 alkyl, for example, selected from methyl and ethyl.

Preferably, in formula A, R₃ is selected from C3-C8 alkyl, more preferably selected from C3-C6 alkyl or C4-C6 alkyl, for example, selected from isopropyl, 1-methylpropyl, 1-methylbutyl, and 1,3-dimethylbutyl.

In some preferred embodiments, in formula A, R₁ and R₂ are each independently selected from C1-C2 alkyl, and R₃ is selected from C3-C6 alkyl, preferably selected from C4-C6 alkyl.

In some embodiments, the compound of formula A is a compound of formula I, a compound of formula II, a compound of formula IV, or a compound of formula VI:

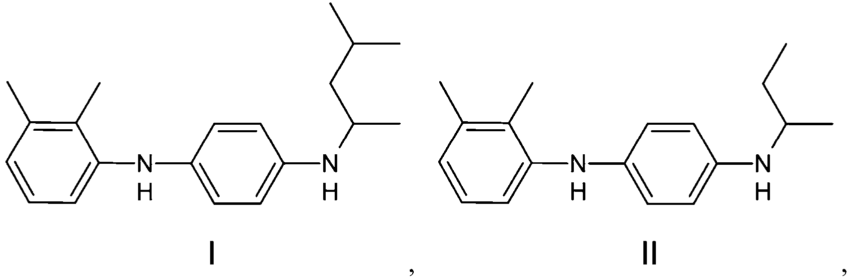

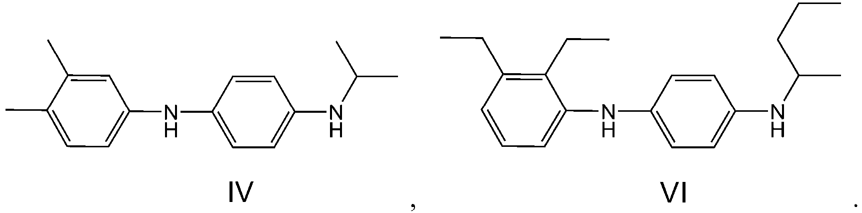

The present invention has found that the use of the compound of formula A in rubber compositions, especially tire rubber compositions, can significantly improve the damp-heat aging resistance and ozone aging resistance of the rubber compositions. The improvement effect is significantly superior to that of traditional p-phenylenediamine antidegradants (such as IPPD), while maintaining good original physical properties and thermal-oxidative aging resistance. The present invention has also found that the combined use of the compound of formula A and antidegradant STMQ in rubber compositions, especially tire rubber compositions, can significantly improve the damp-heat aging resistance and ozone aging resistance of the rubber compositions. The improvement effect is significantly superior to that of the combined use of traditional p-phenylenediamine antidegradants (such as IPPD) and antidegradant STMQ, while maintaining good original physical properties and thermal-oxidative aging resistance.

In the present invention, antidegradant STMQ refers to a polymer of 2,2,4-trimethyl-1,2-dihydroquinoline wherein a total mass fraction of dimers, trimers, and tetramers of 2,2,4-trimethyl-1,2-dihydroquinoline is ≥ 80%. A polymer of 2,2,4-trimethyl-1,2-dihydroquinoline polymer (also known as antidegradant TMQ) has the structure represented by formula B: 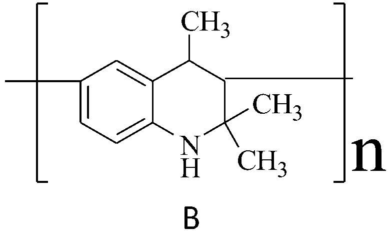 in formula B, n represents the degree of polymerization.

In the present invention, a compound of formula A may be prepared by a method comprising the following steps:
(1) performing a condensation reaction of a compound of formula C with nitrobenzene in the presence of a first catalyst to obtain a condensate comprising a compound of formula D and/or a compound of formula D', followed by carrying out a reduction reaction of the condensate in the presence of H₂ and a second catalyst to obtain a compound of formula E; 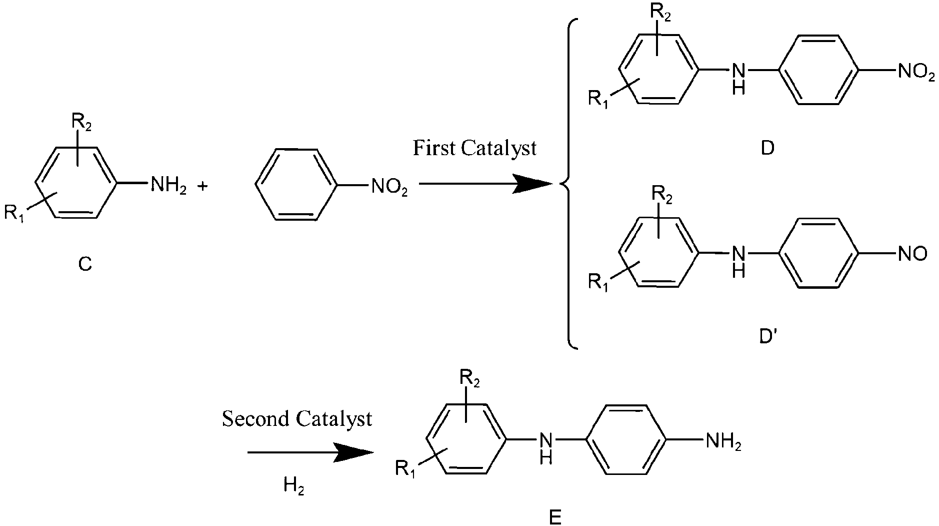
(2) conducting a reductive alkylation reaction of a compound of formula E with a compound of formula F in the presence of H₂ and a third catalyst to obtain a compound of formula A; 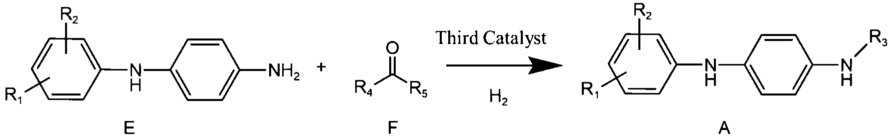
in formula A, formula C, formula D, and formula E, R₁, R₂, and R₃ are as described in any embodiment herein; in formula F, R₄ and R₅ are each independently selected from H and C1-C7 alkyl. Those skilled in the art can determine the suitable R₄ and R₅ in formula F based on R₃ contained in a compound of formula A.

The first catalyst used in step (1) may be one or more selected from the group consisting of alkali metal hydroxides, alkali metal alkoxides, quaternary ammonium hydroxides, and combinations of alkali metal hydroxides with tetraalkylammonium halides. Alkali metal hydroxides suitable for the present invention include sodium hydroxide, potassium hydroxide, lithium hydroxide, and the like. Alkali metal alkoxides suitable for the present invention include sodium methoxide, potassium methoxide, sodium ethoxide, potassium ethoxide, sodium tert-butoxide, potassium tert-butoxide, sodium tert-pentoxide, potassium tert-pentoxide, and the like. Quaternary ammonium hydroxides are a class of compounds with a general formula of R¹₄NOH, wherein R₁ represents four identical or different aliphatic hydrocarbon groups or aromatic hydrocarbon groups. R₁ in the quaternary ammonium hydroxides suitable for the present invention may be one or more selected from methyl, ethyl, propyl, butyl, and the like. Examples of quaternary ammonium hydroxides suitable for the present invention include tetramethylammonium hydroxide, tetraethylammonium hydroxide, tetrabutylammonium hydroxide, and the like. The first catalyst may also be a combination of an alkali metal hydroxide and a tetraalkylammonium halide. Tetraalkylammonium halides have the general formula R²₄NX, where R₂ represents four identical or different aliphatic hydrocarbon groups or aromatic hydrocarbon groups, e.g., methyl, ethyl, propyl, butyl, etc., and X represents a halogen atom, e.g., fluorine, chlorine, bromine, iodine. Examples of combinations of alkali metal hydroxides and tetraalkylammonium halides include sodium hydroxide and tetrabutylammonium bromide, and the like.

A molar ratio of a first catalyst to a compound of formula C may be 0.1:1 to 2:1, preferably 0.9:1 to 1.1:1, for example, 1.05:1, 1.1:1, and 1.5:1. In some embodiments, in step (1), first, a compound of formula C is subjected to form a salt with a first catalyst, and then nitrobenzene is added dropwise to carry out a condensation reaction.

In step (1), a condensate obtained from a condensation reaction of a compound of formula C with nitrobenzene in the presence of a first catalyst may be one or both of a nitro compound represented by formula D and a nitroso compound represented by formula D', and may further contain an azobenzene compound.

In step (1), a molar ratio of a compound of formula C to nitrobenzene may be 2:1 to 15:1, preferably 4:1 to 10:1, more preferably 5:1 to 8:1, for example, 6:1, and 7:1.

In step (1), a condensation reaction may be carried out at a temperature of 40°C to 90°C, preferably 65°C to 85°C; for example, a reaction temperature may be 60°C, 70°C, 75°C, or 80°C. The condensation reaction shall be carried out under vacuum conditions, with a pressure range of -0.09 MPa to -0.1 MPa.

The second catalyst used in step (1) may be a porous metal catalyst or a supported metal catalyst. Porous metal catalysts are also known as spongy metal catalysts. Porous metal catalysts suitable for the present invention include Raney nickel (also known as skeletal nickel), Raney cobalt, Raney copper, and the like. Supported metal catalysts comprise a metal as the catalytically active center and a support for loading the metal. The metal in the supported metal catalysts suitable for the present invention may be nickel, cobalt, copper, platinum, palladium, ruthenium, rhodium, or the like; the support may be carbon, alumina, silica gel, molecular sieve, or the like, and the carbon used as the support may be activated carbon. A molar ratio of the metal in a second catalyst to a condensate may be 0.0001:1 to 0.2:1.

In step (1), a condensate generated from a condensation reaction undergoes a hydrogenation reduction reaction in the presence of a second catalyst to produce a compound of formula E. In step (1), a reduction reaction may be carried out at a temperature of 40°C to 120°C, preferably 60°C to 90°C; for example, a reaction temperature may be 70°C, 75°C, and 80°C. A pressure of hydrogen in a reduction reaction may be 0.5 MPa to 5 MPa, for example, 1 MPa, 1.5 MPa, 2 MPa, and 2.5 MPa.

In step (1), 2,3-dimethylaniline itself may be used as a solvent, or solvents such as toluene and xylene may also be used. After the completion of the reaction in step (1), the reaction solution is subjected to filtration, water washing, and phase separation; an organic phase is then subjected to vacuum distillation to remove light components, thereby obtaining a compound of formula E.

The third catalyst used in Step (2) may be the aforementioned supported metal catalyst, such as Pt/C. A molar ratio of the metal in a third catalyst to a compound of formula E may be 0.0001:1 to 0.2:1.

In step (2), the carbon atom of the carbonyl in a compound of formula F is linked to the nitrogen atom of the amino in a compound of formula E after the reaction. Therefore, a suitable compound of formula F can be selected for the reaction according to the R₃ group contained in a compound of formula A to be prepared. A molar ratio of a compound of formula F to a compound of formula E may be 1:1 to 15:1, for example, 2:1, 3:1, 5:1, 8:1, and 10:1. A reaction temperature of step (2) may be 40°C to 150°C, for example, 50°C, 80°C, 100°C, and 120°C. A pressure of hydrogen in step (2) may be 0.5 MPa to 5 MPa, for example, 1 MPa, 1.5 MPa, 2 MPa, and 2.5 MPa.

In step (2), a compound of formula F, serving as a raw material of a reaction, may be used as the solvent. After the completion of the reaction in step (2), a reaction solution is subjected to filtration and vacuum distillation to remove light components, thereby obtaining a compound of formula A.

In the present invention, liquid chromatography (LC) or gas chromatography (GC) may be used to determine whether each step of the reaction has reached the endpoint, so as to confirm the appropriate reaction time.

An antidegradant composition of the present invention comprises (1) one or more compounds of formula A, and (2) antidegradant STMQ. In some embodiments, an antidegradant composition of the present invention consists of a compound of formula A and antidegradant STMQ. In an antidegradant composition of the present invention, a mass ratio of a compound of formula A to antidegradant STMQ may be 1:1 to 3:1, for example, 1.5:1, 2:1, and 2.5:1.

The raw materials of a rubber composition usually comprise a diene elastomer, a reinforcing filler, an antidegradant, and a crosslinking agent. Herein, a rubber composition includes unvulcanized rubber and vulcanized rubber. Unvulcanized rubber can be converted into vulcanized rubber through vulcanization (curing).

The raw materials of the rubber composition of the present invention comprise a diene elastomer and one or more compounds of formula A. Based on 100 parts by mass of the diene elastomer, an amount of a compound of formula A may be 1 to 5 parts by mass, preferably 1 to 3 parts by mass, for example, 1.5 parts by mass, 2 parts by mass, and 2.5 parts by mass. Herein, unless otherwise specified, the amounts of other components in the raw materials of the rubber composition are calculated based on 100 parts by mass of the diene elastomer in the raw materials of the rubber composition.

In some embodiments, the raw materials of the rubber composition of the present invention comprise a diene elastomer and an antidegradant composition of the present invention. An amount of an antidegradant composition of the present invention may be 1 to 5 parts by mass, for example, 1.5 parts by mass, 2 parts by mass, 2.5 parts by mass, 3 parts by mass, 3.5 parts by mass, and 4 parts by mass.

Herein, a diene elastomer refers to an elastomer whose monomers comprise dienes (e.g., butadiene, isoprene). The diene elastomers suitable for the present invention may be various diene elastomers known in the art, including but not limited to one or more selected from natural rubber (NR), butadiene rubber (BR), polyisoprene rubber, styrene-butadiene rubber (SBR), chloroprene rubber (CR), nitrile-butadiene rubber (NBR), isoprene/butadiene copolymers, isoprene/styrene copolymers, and isoprene/butadiene/styrene copolymers. In some preferred embodiments, the diene elastomer comprises or consists of natural rubber and butadiene rubber. The mass ratio of natural rubber to butadiene rubber is preferably 1:2 to 2:1, for example, 1:1.5 to 1.5:1, 4.5:5.5 to 5.5:4.5, and 1:1.

The antidegradant contained in the rubber composition of the present invention comprises a compound of formula A. In the raw materials of the rubber composition of the present invention, the amount of the compound of formula A may be 1 to 5 parts by mass, preferably 1 to 3 parts by mass, for example, 1.5 parts by mass, 2 parts by mass, and 2.5 parts by mass. Controlling the amount of a compound of formula A within the aforementioned range is conducive to ensuring the effect of improving damp-heat aging resistance and ozone aging resistance at a relatively low dosage.

In some embodiments, the raw materials of the rubber composition of the present invention comprise the compound of formula A and antidegradant STMQ, i.e., comprising the antidegradant composition of the present invention. In the raw materials of the rubber composition of the present invention, the amount of the antidegradant composition of the present invention may be 1 to 5 parts by mass, for example, 1.5 parts by mass, 2 parts by mass, 2.5 parts by mass, 3 parts by mass, 3.5 parts by mass, and 4 parts by mass; among them, the amount of antidegradant STMQ is preferably 0.5 to 2 parts by mass, for example, 0.8 parts by mass, 1 part by mass, 1.2 parts by mass, and 1.5 parts by mass, and the amount of the compound of formula A is preferably 1 to 3 parts by mass, for example, 1.5 parts by mass, 2 parts by mass, and 2.5 parts by mass. Controlling the amount of the antidegradant composition of the present invention within the aforementioned range is conducive to ensuring the effect of improving damp-heat aging resistance and ozone aging resistance at a relatively low dosage.

In some embodiments, the antidegradant contained in the raw materials of the rubber composition of the present invention only comprises the compound of formula A, or only comprises the compound of formula A and antidegradant STMQ.

The raw materials of the rubber composition of the present invention may comprise a reinforcing filler. In the raw materials of the rubber composition of the present invention, the amount of the reinforcing filler may be 30 to 70 parts by mass, for example, 40 parts by mass, 45 parts by mass, 50 parts by mass, 55 parts by mass, and 60 parts by mass. The reinforcing fillers suitable for the present invention may be conventional reinforcing fillers used in rubber compositions, including but not limited to one or more selected from carbon black, silica, titanium oxide, calcium carbonate, magnesium carbonate, aluminum hydroxide, magnesium hydroxide, clay, and talc. In some preferred embodiments, the reinforcing filler comprises carbon black, or the reinforcing filler is carbon black.

The raw materials of the rubber composition of the present invention comprise a crosslinking agent, such as sulfur. In the raw materials of the rubber composition of the present invention, the amount of sulfur may be 0.5 to 3 parts by mass, for example, 1 part by mass, 1.5 parts by mass, 2 parts by mass, and 2.5 parts by mass.

The raw materials of the rubber composition of the present invention may further comprise other components commonly used in rubber compositions, including but not limited to one or more selected from softeners, protective waxes, activators, and accelerators.

Softeners can be used to improve the processing performance of the rubber composition. Softeners may comprise petroleum-based softeners, such as naphthenic oil, aromatic oil, processing oil, lubricating oil, paraffin, liquid paraffin, petroleum asphalt, petrolatum, and the like; they may also comprise fatty oil softeners, such as stearic acid, castor oil, linseed oil, rapeseed oil, coconut oil, waxes (e.g., beeswax, carnauba wax, and lanolin), tall oil, linoleic acid, palmitic acid, lauric acid, and the like. In the raw materials of the rubber composition of the present invention, the amount of the softener may be 2 to 15 parts by mass, for example, 3 parts by mass, 5 parts by mass, 7 parts by mass, 8 parts by mass, 9 parts by mass, 10 parts by mass, 11 parts by mass, and 13 parts by mass. In some preferred embodiments, the softener comprises or consists of aromatic oil and stearic acid. The mass ratio of aromatic oil to stearic acid may be 1:1 to 5:1, for example, 2:1, 3:1, 3.5:1, and 4:1. In some preferred embodiments, in the raw materials of the rubber composition of the present invention, the amount of aromatic oil is 1 to 10 parts by mass, for example, 5 parts by mass, 6 parts by mass, 7 parts by mass, 8 parts by mass, and 9 parts by mass; the amount of stearic acid is 1 to 5 parts by mass, for example, 1.5 parts by mass, 2 parts by mass, and 3 parts by mass.

Protective wax can migrate from the interior of the rubber to the surface of the rubber to form a wax film, functioning to isolate the rubber of the surface from the external environment. Protective wax is optionally added. When the raw materials of the rubber composition of the present invention comprise protective wax, the amount of protective wax may be 1 to 5 parts by mass, for example, 1.5 parts by mass, 2 parts by mass, 3 parts by mass, and 4 parts by mass.

Activators can function to accelerate the vulcanization rate, enhance the thermal conductivity, wear resistance, and tear resistance of the rubber. In the raw materials of the rubber composition of the present invention, the amount of the activator may be 1 to 10 parts by mass, for example, 2 parts by mass, 3 parts by mass, 4 parts by mass, 5 parts by mass, 6 parts by mass, 7 parts by mass, 8 parts by mass, and 9 parts by mass. In some preferred embodiments, the activator comprises ZnO, or the activator is ZnO. In some preferred embodiments, in the raw materials of the rubber composition of the present invention, the amount of ZnO is 3 to 8 parts by mass, for example, 4 parts by mass, 5 parts by mass, 6 parts by mass, and 7 parts by mass.

Accelerators usually refer to vulcanization accelerators, and may be selected from one or more of sulfonamide vulcanization accelerators, thiazole vulcanization accelerators, thiuram vulcanization accelerators, thiourea vulcanization accelerators, guanidine vulcanization accelerators, dithiocarbamate vulcanization accelerators, aldehyde-amine vulcanization accelerators, aldehyde-ammonia vulcanization accelerators, imidazoline vulcanization accelerators, and xanthate acid vulcanization accelerators. In the raw materials of the rubber composition of the present invention, the amount of the accelerator may be 0.2 to 2 parts by mass, for example, 0.5 parts by mass, 0.6 parts by mass, 0.8 parts by mass, 1 part by mass, and 1.5 parts by mass. In some preferred embodiments, the accelerator is accelerator NS (N-tert-butylbenzothiazole-2-sulphenamide).

In addition, when necessary, a plasticizer may also be used in the rubber composition, such as DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate), and the like. The dosage of the plasticizer may be a conventional dosage in the art.

In some preferred embodiments, the raw materials of the rubber composition of the present invention comprise: 100 parts by mass of a diene elastomer, 0.5 to 3 parts by mass of sulfur, 1 to 5 parts by mass of the compound of formula A or the antidegradant composition of the present invention, 30 to 70 parts by mass of carbon black, 1 to 10 parts by mass of ZnO, 1 to 5 parts by mass of stearic acid, 1 to 10 parts by mass of aromatic oil, and 0.2 to 2 parts by mass of an accelerator, or consist of the above components; wherein the diene elastomer preferably comprises natural rubber and butadiene rubber in a mass ratio of 1:2 to 2:1, and the accelerator is preferably accelerator NS.

The unvulcanized rubber of the present invention may be prepared by conventional rubber mixing methods, for example, by a two-stage mixing process: first-stage thermomechanical (e.g., internal mixer) mixing, mixing the raw materials of the rubber composition excluding the crosslinking agent and accelerator, and kneading the entire mixture until a maximum temperature between 110°C and 190°C is reached to obtain the first-stage rubber; second-stage thermomechanical (e.g., open mill) mixing, after cooling the first-stage rubber, mixing the first-stage rubber with the crosslinking agent and accelerator until a maximum temperature below 110°C is reached to obtain the second-stage rubber, i.e., the unvulcanized rubber.

Unvulcanized rubber can be vulcanized (cured) to obtain vulcanized rubber. The temperature of vulcanization is usually 130°C to 200°C, for example, 140°C to 160°C or 145°C ± 5°C; the time for vulcanization depends on the temperature of vulcanization, vulcanization system, and vulcanization kinetics, and is usually 15 to 60 minutes, for example, 20 to 40 minutes and 30 minutes ± 5 minutes.

The rubber composition of the present invention is used in rubber products, especially tires. Compared with the use of traditional p-phenylenediamine antidegradants, such as IPPD, the use of the compound of formula A or the antidegradant composition of the present invention can significantly improve the damp-heat aging resistance and ozone aging resistance of rubber products. Therefore, the present invention also provides a rubber product containing the rubber composition described herein. The rubber product may be a tire, a rubber overshoe, a sealing strip, an acoustic panel, or a crash pad, etc. The rubber product is preferably a tire, such as a sidewall rubber, a tread rubber, or the like.

The present invention further provides the use of a compound of formula A or an antidegradant composition of the present invention in improving the damp-heat aging resistance and/or ozone aging resistance of a rubber composition, as well as a method for improving the damp-heat aging resistance and/or ozone aging resistance of a rubber composition. Preferably, the use or method of the present invention comprises: adding 1 to 5 parts by mass of the compound of formula A or the antidegradant composition of the present invention to the raw materials of the rubber composition, based on 100 parts by mass of the diene elastomer contained in the rubber composition. In the use or method of the present invention, the raw material composition of the rubber composition is preferably as described in any embodiment herein.

The present invention will be illustrated by way of specific examples below. It should be understood that these examples are merely explanatory and is not intended to limit the scope of the present invention. Unless otherwise specified, the methods, reagents, and materials used in the examples are conventional methods, reagents, and materials in the art. The formulations used in the examples are commercially available.

The raw materials used in the examples are from the following sources: natural rubber (SCR5), Xishuangbanna Sinochem Rubber Co., Ltd.; butadiene rubber (BR9000), Shandong Yuhuang Chemical Co., Ltd.; antidegradant 6PPD, Sennics Co., Ltd.; antidegradant STMQ, Kemai Chemical Co., Ltd.; carbon black N550, zinc oxide, stearic acid, aromatic oil, sublimed sulfur and accelerator NS are all common raw materials in the rubber industry.

### Preparation Example 1: synthesis of the compound of formula I

### (1) Synthesis of the compound of formula III

181.5g (1.5mol) of 2,3-dimethylaniline and 100.1g (0.275mol) of a 25% aqueous solution of tetramethylammonium hydroxide (TMAOH) are added to a 500 mL four-necked flask. The mixture is stirred and heated to 40-50°C and then dehydrated by vacuum distillation to allow TMAOH to form a salt with 2,3-dimethylaniline. During this process, the reaction solution gradually turns from yellow to deep red. The temperature is gradually increased to 72°C, when the fraction is approximately 50% of the feeding amount of the 25% TMAOH catalyst, 31g (0.25mol) of nitrobenzene is added dropwise during distillation at 72°C under reduced pressure (-0.098 MPa) over a period of about 3h. After the addition is complete, the solution is kept at this temperature for 1 h. The reaction is monitored by LC until the nitrobenzene is completely reacted, yielding a condensate.

The above condensate is transferred to a 500mL stainless steel reactor, 50g of deionized water and 40g of skeletal nickel catalyst are added, the reactor is purged with hydrogen three times, heated to 75°C, and pressurized to 1.5MPa for reaction. LC monitoring is performed until the nitro and nitroso compounds are completely reduced. The reaction solution is filtered, washed with water, and separated into phases. The organic phase is distilled under reduced pressure (-0.1MPa, 180°C) to remove light components, yielding 45.7g of compound III (yield is approximately 85%), with a GC-detected content >96%.

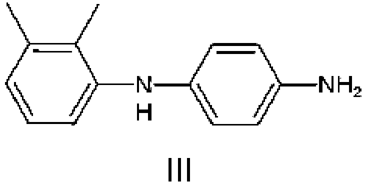

LC-MS(m/z): 212.30(M-H⁺).

### (2) Synthesis of the compound of formula I

42.5g (0.2mol) of compound III, 60g (0.6mol) of 4-methyl-2-pentanone, and 0.5g of Pt/C catalyst are added to a reactor. The reactor is purged with hydrogen three times, heated to 100°C, and pressurized to 1.5MPa. The reaction is stopped, as detected by GC, when the content of compound III is <0.1%. The mixture is cooled, filtered to remove the catalyst, and distilled at - 0.1MPa and 180°C to remove light components, yielding 57.6g of compound I (yield is approximately 97.2%), with a GC-detected content >96.8%. Appearance: Reddish-brown liquid.

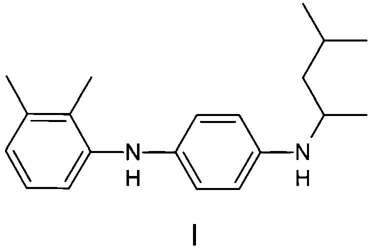

LC-MS(m/z): 296.46(M-H⁺).

### Preparation Example 2: synthesis of the compound of formula II

### (1) Synthesis of the compound of formula III

The synthesis of compound III is the same as in Preparation Example 1.

### (2) Synthesis of the compound of formula II

42.5g (0.2mol) of compound III, 100g (1.38mol) of 2-butanone, and 0.6g of Pt/C catalyst are added to a reactor. The reactor is purged with hydrogen three times, heated to 80°C, and pressurized to 1.5MPa. The reaction is stopped, as detected by GC, when the content of compound III is <0.1%. The mixture is cooled, filtered to remove the catalyst, and distilled at -0.1 MPa and 150°C to remove light components, yielding 52.2g of compound II (yield is approximately 97.4%) with a GC-detected content >95.8%. Appearance: Reddish-brown solid.

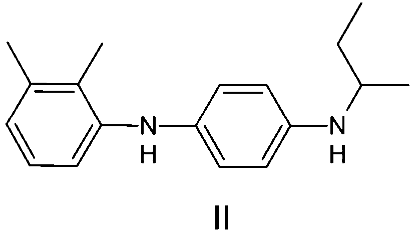

LC-MS(m/z): 268.40(M-H⁺).

### Preparation Example 3: synthesis of the compound of formula IV

### (1) Synthesis of the compound of formula V

181.5g (1.5mol) of 3,4-dimethylaniline and 100.1g (0.275mol) of a 25% aqueous solution of tetramethylammonium hydroxide (TMAOH) are added to a 500 mL four-necked flask. The mixture is stirred and heated to 40-50°C and then dehydrated by vacuum distillation to allow TMAOH to form a salt with 2,3-dimethylaniline. During this process, the reaction solution gradually turns from yellow to deep red. The temperature is gradually increased to 72°C, when the fraction is approximately 50% of the feeding amount of the 25% TMAOH catalyst, 31g (0.25mol) of nitrobenzene is added dropwise during distillation at 72°C under reduced pressure (-0.098 MPa) over a period of about 3h. After the addition is complete, the solution is kept at this temperature for 1h. The reaction is monitored by LC until the nitrobenzene is completely reacted, yielding a condensate.

The above condensate is transferred to a 500 mL stainless steel reactor, 50g of deionized water and 40g of skeletal nickel catalyst are added, the reactor is purged with hydrogen three times, heated to 75°C, and pressurized to 1.5MPa for reaction. LC monitoring is performed until the nitro and nitroso compounds are completely reduced. The reaction solution is filtered, washed with water, and separated into phases. The organic phase is distilled under reduced pressure (-0.1MPa, 180°C) to remove light components, yielding 43.5g of compound V (yield is approximately 82%), with a GC-detected content >95%.

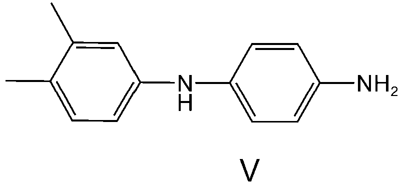

LC-MS(m/z): 212.30(M-H⁺).

### (2) Synthesis of the compound of formula IV

42.5g (0.2mol) of compound V, 162.4 g (2.8mol) of acetone, and 0.5g of Pt/C catalyst are added to a reactor. The reactor is purged with hydrogen three times, heated to 70°C, and pressurized to 1.5MPa. The reaction is stopped, as detected by GC, when the content of compound V is <0.1%. The mixture is cooled, filtered to remove the catalyst, and distilled at -0.1MPa and 180°C to remove light components, yielding 49g of compound IV (yield is approximately 96.5%), with a GC-detected content >96.0%. Appearance: Reddish-brown solid.

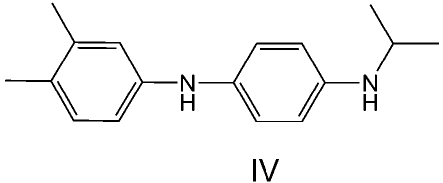

LC-MS(m/z): 254.38(M-H⁺).

### Preparation Example 4: synthesis of the compound of formula VI

### (1) Synthesis of the compound of formula VII

223.8g (1.5mol) of 2,3-diethylaniline and 100.1g (0.275mol) of 25% aqueous solution of tetramethylammonium hydroxide (TMAOH) are added to a 500 mL four-necked flask. The mixture is stirred and heated to 40-50°C and then dehydrated by vacuum distillation to allow TMAOH to form a salt with 2,3-dimethylaniline. During this process, the reaction solution gradually turns from yellow to deep red. The temperature is gradually increased to 72 °C, when the fraction is approximately 50% of the feeding amount of the 25% TMAOH catalyst, 31g (0.25mol) of nitrobenzene is added dropwise during distillation at 72°C under reduced pressure (-0.098 MPa) over a period of about 3h. After the addition is complete, the solution is kept at this temperature for 1 h. The reaction is monitored by LC until the nitrobenzene is completely reacted, yielding a condensate.

The above condensate is transferred to a 500mL stainless steel reactor, 50g of deionized water and 40g of skeletal nickel catalyst are added, the reactor is purged with hydrogen three times, heated to 75°C, and pressurized to 1.5MPa for reaction. LC monitoring is performed until the nitro and nitroso compounds are completely reduced. The reaction solution is filtered, washed with water, and separated into phases. The organic phase is distilled under reduced pressure (-0.1MPa, 180°C) to remove light components, yielding 45.6g of compound VII (yield is approximately 75.9%), with a GC-detected content >94.5%.

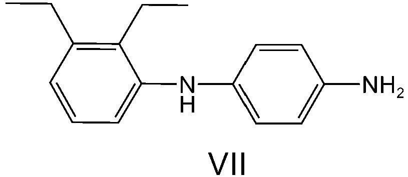

LC-MS(m/z): 240.35(M-H⁺).

### (2) Synthesis of the compound of formula VI

43.2g (0.18mol) of compound VII, 155g (1.8mol) of 2-pentanone, and 0.5g of Pt/C catalyst are added to a reactor. The reactor is purged with hydrogen three times, heated to 100°C, and pressurized to 1.5MPa. The reaction is stopped, as detected by GC, when the content of compound VII is <0.1%. The mixture is cooled, filtered to remove the catalyst, and distilled at - 0.1 MPa and 180°C to remove light components, yielding 53.2g of compound VI (yield is approximately 95.3%) with a GC-detected content >96.8%. Appearance: Reddish-brown liquid.

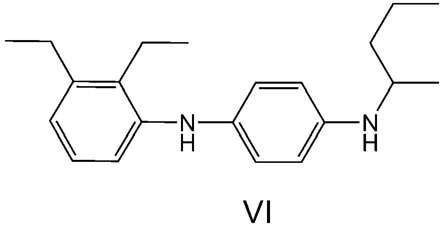

LC-MS(m/z): 310.49(M-H⁺).

### Examples 1-6 and Comparative Examples 1-3

The rubber compositions of Examples 1-6 and Comparative Examples 1-3 are prepared using the following process according to the formulations shown in Table 1:
(1) Natural rubber and butadiene rubber are added to an internal mixer; subsequently, carbon black, zinc oxide, stearic acid, aromatic oil and antidegradants (antidegradant IPPD, a compound of formula I, a compound of formula II, a compound of formula IV, a compound of formula VI, and/or antidegradant STMQ) are added. The mixture is kneaded until the temperature of the rubber composition reaches 130°C and then discharged to obtain a first-stage rubber.
(2) After the first-stage rubber is cooled, it is milled on an open mill, accelerator NS and sulfur are added, and the mixture is kneaded until the temperature of the rubber composition reaches 70°C and then sheeted out to obtain the second-stage rubber.
(3) The second-stage rubber is vulcanized (145°C×30min) to obtain vulcanized rubber.

**Table 1: Formulation of Rubber Composition (unit: parts by mass)**

| Material | Compar ative Example 1 | Compar ative Example 2 | Compar ative Example 3 | Example 1 | Example 2 | Example 3 | Example 4 | Example 5 | Example 6 |
|---|---|---|---|---|---|---|---|---|---|
| SCR5 | 50 | 50 | 50 | 50 | 50 | 50 | 50 | 50 | 50 |
| BR9000 | 50 | 50 | 50 | 50 | 50 | 50 | 50 | 50 | 50 |
| Carbon Black N550 | 50 | 50 | 50 | 50 | 50 | 50 | 50 | 50 | 50 |
| Zinc oxide | 5 | 5 | 5 | 5 | 5 | 5 | 5 | 5 | 5 |
| Stearic acid | 2 | 2 | 2 | 2 | 2 | 2 | 2 | 2 | 2 |
| Aromatic oil | 7 | 7 | 7 | 7 | 7 | 7 | 7 | 7 | 7 |
| Antidegradant IPPD | 2 | 2 | 1.5 | 0 | 0 | 0 | 0 | 0 | 0 |
| A compound of formula I | 0 | 0 | 0 | 2 | 0 | 0 | 0 | 1.5 | 2 |
| A compound of formula II | 0 | 0 | 0 | 0 | 2 | 0 | 0 | 0 | 0 |
| A compound of formula IV | 0 | 0 | 0 | 0 | 0 | 2 | 0 | 0 | 0 |
| A compound of formula VI | 0 | 0 | 0 | 0 | 0 | 0 | 2 | 0 | 0 |
| Antidegradant STMQ | 1 | 0 | 1.5 | 1 | 1 | 1 | 1 | 1.5 | 0 |
| Sublimed sulfur | 1.5 | 1.5 | 1.5 | 1.5 | 1.5 | 1.5 | 1.5 | 1.5 | 1.5 |
| Accelerator NS | 0.8 | 0.8 | 0.8 | 0.8 | 0.8 | 0.8 | 0.8 | 0.8 | 0.8 |
| Total | 168.3 | 167.3 | 167.3 | 168.3 | 168.3 | 168.3 | 168.3 | 168.3 | 167.3 |

### Test Example 1: High-Temperature Water Immersion Test

The vulcanized rubbers of Examples 1-6 and Comparative Examples 1-3 are immersed in deionized water at 90°C for 8 hours. The soaking solution is then evaporated and dried, and the soaked precipitate is subsequently dissolved in acetone and subjected to liquid chromatography analysis to determine the content of the antidegradant therein. The results are shown in Tables 2-4.

**Table 2: Results of High-Temperature Water Immersion Test of vulcanized rubber**

| | Comparative Example 1 | Example 1 | Example 2 | Example 3 | Example 4 |
|---|---|---|---|---|---|
| Mass of the antidegradant in the soaked precipitate (mg/100 g rubber) | 0.724 | 0.092 | 0.130 | 0.145 | 0.083 |
| Variation relative to Comparative Example 1 | / | -87% | -82% | -80% | -89% |

**Table 3: Results of High-Temperature Water Immersion Test of vulcanized rubber**

| | | |
|---|---|---|
| | Comparative Example 2 | Example 6 |
| Mass of the antidegradant in the soaked precipitate (mg/100 g rubber) | 0.705 | 0.100 |
| Variation relative to Comparative Example 2 | / | -86% |

**Table 4: Results of High-Temperature Water Immersion Test of vulcanized rubber**

| | Comparative Example 3 | Example 5 |
|---|---|---|
| Mass of the antidegradant in the soaked precipitate (mg/100 g rubber) | 0.557 | 0.079 |
| Variation relative to Comparative Example 2 | / | -86% |

### Test Example 2: Damp-Heat Aging Test

In accordance with GB/T 15905-1995 Test Method for Damp-Heat Aging of Vulcanized Rubber, the high temperature and high humidity aging resistance test is conducted on the vulcanized rubbers of Examples 1-6 and Comparative Examples 1-3. The test conditions are an aging oven temperature of 70°C, and a relative humidity of 90%, with an aging time of 14 days. The test standards for elongation at break and tensile strength are GB/T 528-2009 Determination of Tensile Stress-Strain Properties of Vulcanized Rubber or Thermoplastic Rubber. The results are shown in Tables 5-7.

Herein, Tensile Product = Tensile Strength × Elongation at Break.

Herein, Retention Rate of Physical Properties = Tensile Product after Aging / Tensile Product before Aging.

**Table 5: Physical property results of vulcanized rubber before and after high temperature and high humidity aging**

| | Test project | Comparative Example 1 | Example 1 | Example 2 | Example 3 | Example 4 |
|---|---|---|---|---|---|---|
| Physical properties before Aging | Tensile strength /MPa | 19.3 | 19.2 | 19.2 | 19.2 | 19.3 |
| | Elongation at break /% | 550 | 545 | 555 | 540 | 550 |
| | Tensile product | 10615 | 10464 | 10656 | 10368 | 10615 |
| Physical properties after Aging | Tensile strength /MPa | 16.0 | 17.1 | 17.0 | 16.8 | 17.1 |
| | Elongation at break /% | 460 | 490 | 495 | 485 | 495 |
| | Tensile product | 7675 | 8379 | 8415 | 8148 | 8465 |
| | Retention rate of physical properties/% | 72.3 | 80.1 | 79.0 | 79.0 | 79.7 |

**Table 6: Physical property results of vulcanized rubber before and after high temperature and high humidity aging**

| | Test project | Comparative Example 2 | Example 6 |
|---|---|---|---|
| Physical properties before Aging | Tensile strength /MPa | 19.4 | 19.3 |
| | Elongation at break /% | 560 | 555 |
| | Tensile product | 10864 | 10711 |
| Physical properties after Aging | Tensile strength /MPa | 15.0 | 15.7 |
| | Elongation at break /% | 425 | 455 |
| | Tensile product | 6375 | 7144 |
| | Retention rate of physical properties/% | 59% | 67% |

**Table 7: Physical property results of vulcanized rubber before and after high temperature and high humidity aging**

| | Test project | Comparative Example 3 | Example 5 |
|---|---|---|---|
| Physical properties before Aging | Tensile strength /MPa | 19.2 | 19.3 |
| | Elongation at break /% | 550 | 545 |
| | Tensile product | 10560 | 10519 |
| Physical properties after Aging | Tensile strength /MPa | 16.3 | 17.4 |
| | Elongation at break /% | 475 | 500 |
| | Tensile product | 7743 | 8700 |
| | Retention rate of physical properties/% | 73% | 83% |

### Test Example 3: Thermal-Oxidative Aging Test

In accordance with GB/T 3512-2014 Accelerated Aging and Heat Resistance Test for Vulcanized Rubber or Thermoplastic Rubber by Hot Air, the thermal-oxidative aging test is conducted on the vulcanized rubbers of Examples 1-6 and Comparative Examples 1-3. The test condition is 100°C × 48 hours. The test standards for elongation at break and tensile strength are GB/T 528-2009 Determination of Tensile Stress-Strain Properties of Vulcanized Rubber or Thermoplastic Rubber. The results are shown in Tables 8-10.

**Table 8: Physical property results of vulcanized rubber before and after hot air aging**

| | Test project | Comparative Example 1 | Example 1 | Example 2 | Example 3 | Example 4 |
|---|---|---|---|---|---|---|
| Physical properties before Aging | Tensile strength /MPa | 19.3 | 19.2 | 19.2 | 19.2 | 19.3 |
| | Elongation at break /% | 550 | 545 | 555 | 540 | 550 |
| | Tensile product | 10615 | 10464 | 10656 | 10368 | 10615 |
| Physical properties after Aging | Tensile strength /MPa | 17.5 | 17.3 | 17.4 | 17.6 | 17.2 |
| | Elongation at break /% | 490 | 490 | 485 | 485 | 495 |
| | Tensile product | 8575 | 8477 | 8439 | 8536 | 8514 |
| | Retention rate of physical properties/% | 80.8 | 81.0 | 79.2 | 82.3 | 80.2 |

**Table 9: Physical property results of vulcanized rubber before and after hot air aging**

| | Test project | Comparative Example 2 | Example 6 |
|---|---|---|---|
| Physical properties before Aging | Tensile strength /MPa | 19.4 | 19.3 |
| | Elongation at break /% | 560 | 555 |
| | Tensile product | 10864 | 10711 |
| Physical properties after Aging | Tensile strength /MPa | 14.3 | 14.0 |
| | Elongation at break /% | 400 | 410 |
| | Tensile product | 5720 | 5740 |
| | Retention rate of physical properties/% | 52.7% | 53.6% |

**Table 10: Physical property results of vulcanized rubber before and after hot air aging**

| | Test project | Comparative Example 3 | Example 5 |
|---|---|---|---|
| Physical properties before Aging | Tensile strength /MPa | 19.2 | 19.3 |
| | Elongation at break /% | 550 | 545 |
| | Tensile product | 10560 | 10519 |
| Physical | Tensile strength | 17.8 | 17.7 |
| properties after Aging | /MPa | | |
| | Elongation at break /% | 500 | 505 |
| | Tensile product | 8900 | 8939 |
| | Retention rate of physical properties/% | 84.2% | 85.0% |

### Test Example 4: Ozone Aging Test

The ozone aging tests of the vulcanized rubbers of Examples 1-6 and Comparative Examples 1-3 are conducted in an ozone aging test chamber in accordance with the following standards:
Dynamic ozone aging: GB/T 13642-2015 Dynamic Tensile Test for Ozone Cracking Resistance of Vulcanized Rubber or Thermoplastic Rubber.

Static ozone aging: GB/T 7762-2014 Static Tensile Test for Ozone Cracking Resistance of Vulcanized Rubber or Thermoplastic Rubber.

Evaluation of Crack Grade: GB/T 11206-2009 Rubber Aging Tests - Surface Cracking Method.

The specific test parameters are as follows: a volume concentration of ozone is 50 pphm, temperature is (40±2) °C, humidity is (50±5) %. During the static test, the sample is pre-stretched by 20%, and the cracking of the sample is observed at regular intervals. During the dynamic test, the sample is pre-stretched by 10% with a dynamic tensile strain of 10% and a frequency of 0.5 Hz, and the cracking of the sample is observed at regular intervals. The results of dynamic ozone aging and static ozone aging are shown in Table 11 and Table 12, respectively. For the meanings of 1c, 2c, 3c, and 4c in Table 11 and Table 12, refer to GB/T 11206-2019.

**Table 11: Results of Crack Grade of Dynamic Ozone Aging**

| Aging time/h | Compara tive Example 1 | Compara tive Example 2 | Compara tive Example 3 | Example 1 | Example 2 | Example 3 | Example 4 | Example 5 | Example 6 |
|---|---|---|---|---|---|---|---|---|---|
| 2 | 1c | 1c | 1c | 1b | lb | lb | 1b | 1c | 1b |
| 4 | 1c | 1c | 1c | 1b | lb | lb | 1b | 1c | 1b |
| 8 | 1c | 1c | 1c | 1c | lb | 1c | 1c | 1c | 1c |
| 24 | 1c | 1c | 2c | 1c | 1c | 1c | 1c | 1c | 1c |
| 48 | 2c | 2c | 2c | 2c | 2c | 2c | 2c | 2c | 2c |
| 72 | 2c | 2c | 3c | 2c | 2c | 2c | 2c | 2c | 2c |
| 96 | 3c | 3c | 3c | 2c | 2c | 2c | 2c | 3c | 2c |
| 120 | 3c | 4c | 4c | 3c | 2c | 3c | 3c | 3c | 3c |
| 144 | 4c | 4c | 4c | 3c | 3c | 3c | 3c | 4c | 4c |

**Table 12: Results of Crack Grade of Static Ozone Aging**

| Aging time/h | Compara tive Example 1 | Compara tive Example 2 | Compara tive Example 3 | Example 1 | Example 2 | Example 3 | Example 4 | Example 5 | Example 6 |
|---|---|---|---|---|---|---|---|---|---|
| 2 | 1c | 1c | 1c | 1c | 1c | 1c | 1c | 1c | 1c |
| 4 | 1c | 1c | 1c | 1c | 1c | 1c | 1c | 1c | 1c |
| 8 | 1c | 1c | 1c | 1c | 1c | 1c | 1c | 1c | 1c |
| 24 | 1c | 1c | 2c | 1c | 1c | 1c | 1c | 2c | 1c |
| 48 | 2c | 2c | 2c | 2c | 2c | 2c | 2c | 2c | 2c |
| 72 | 2c | 2c | 3c | 2c | 2c | 2c | 2c | 2c | 2c |
| 96 | 3c | 3c | 3c | 2c | 2c | 3c | 3c | 3c | 2c |
| 120 | 4c | 4c | 4c | 3c | 3c | 3c | 3c | 4c | 3c |
| 144 | 4c | 4c | 4c | 3c | 3c | 3c | 3c | 4c | 4c |

The results in Table 2 show that after the rubber compositions of Examples 1-4 are immersed in water at 90°C for 8 hours, the content of antidegradant in the soaked precipitate is significantly lower than that in Comparative Example 1, accounting for less than 20% of that in Comparative Example 1. The results in Table 3 indicate that content of antidegradant in the soaked precipitate of the rubber composition of Example 6 is notably lower than that in Comparative Example 2. As shown in the results of Table 4, the content of antidegradant in the soaked precipitate of the rubber composition of Example 5 is obviously lower than that in Comparative Example 3. The foregoing results demonstrate that the antidegradants in Examples 1-6 do not readily migrate out of the rubber compositions under high-temperature and high-humidity conditions.

The results in Table 8 show that the performance retention rates of the rubber compositions of Examples 1-4 and Comparative Example 1 are at the same level after hot air aging at 100°C for 48 hours, indicating that the compounds of formula I, formula II, formula IV, and formula VI have the same effect as traditional p-phenylenediamine antidegradants in improving the thermal-oxidative aging resistance of the rubber compositions.

However, as shown in Table 5, after the rubber compositions of Examples 1-4 are aged for 14 days under high-temperature and high-humidity conditions with a temperature of 70°C and a relative humidity of 90%, their physical property retention rates maintain at approximately 80%, while the physical property retention rate of the rubber composition of Comparative Example 1 is only around 70%. According to the results in Table 6, the physical property retention rate of the rubber composition of Example 6 after high temperature and high humidity aging is significantly higher than that of Comparative Example 2. As presented in Table 7, the physical property retention rate of the rubber composition of Example 5 after high temperature and high humidity aging is notably higher than that of Comparative Example 3. The foregoing results demonstrate that the antidegradants in the rubber compositions of Examples 1-6 are not easily dissolved and extracted by water under high-temperature and high-humidity conditions, and can still maintain a relatively high concentration in the rubber compositions, thereby exerting a good protective effect and exhibiting excellent high temperature and high humidity aging resistance.

The results in Table 11 show that under dynamic ozone aging conditions, the initial crack density (Grade b) of the rubber sheets of Examples 1-4 is significantly lower than that of the rubber sheet of Comparative Example 1 (Grade c). In terms of the growth rate of crack width, the crack growth of the rubber sheets of Examples 1-4 is notably slower than that of Comparative Example 1. After 144 hours of ozone aging, the crack grade of the rubber sheet of Comparative Example 1 reaches Grade 4, while the crack grades of all rubber sheets of Examples 1-4 remain at Grade 3, exhibiting a significant performance improvement. The earliest aging time required for the crack grade of Examples 1-4 to reach Grade 3 is 120 hours, whereas that of Comparative Example 1 is only 96 hours, with a performance improvement of more than 25%.

The results in Table 12 show that after 120 hours of static ozone aging, the crack grade of the rubber sheet of Comparative Example 1 reaches Grade 4, while the crack grades of the rubber sheets of Examples 1-4 only remain at Grade 3, with a significant performance improvement. When the aging time is further extended to 144 hours, the crack grades of the rubber sheets of Examples 1-4 still maintain at Grade 3, demonstrating good durability.

Based on the above results, the compound of formula A and the antidegradant composition of the present invention enable the rubber composition to exhibit excellent high temperature and high humidity aging resistance and ozone aging resistance on the premise of maintaining the original physical properties and thermal-oxidative aging resistance substantially unchanged.

## Claims

1. A compound of formula A: 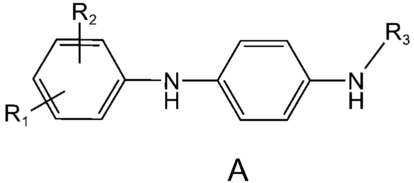 in formula A, R₁, R₂ and R₃ are each independently selected from C1-C8 alkyl;
the compound of formula A excludes a compound that R₁ and R₂ are methyl and R₃ is an isopropyl.

2. The compound of formula A of claim 1, wherein in formula A, R₁ and R₂ are each independently selected from C1-C2 alkyl, and R₃ is selected from C3-C6 alkyl, preferably selected from C4-C6 alkyl.

3. The compound of formula A of claim 1, wherein the compound of formula A is a compound of formula I, a compound of formula II, or a compound of formula VI:

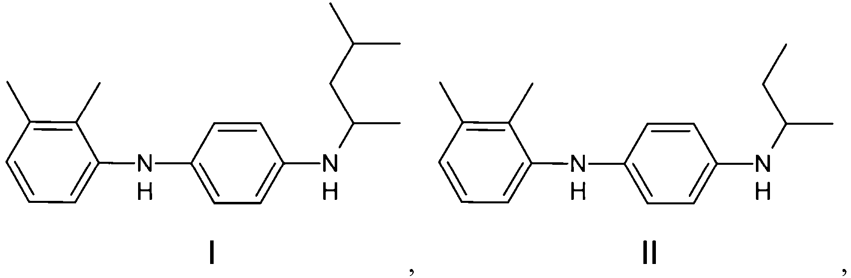

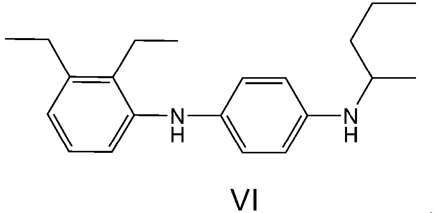

4. An antidegradant composition, wherein the antidegradant composition comprises antidegradant STMQ and a compound of formula A, the antidegradant STMQ is a polymer of 2,2,4-trimethyl-1,2-dihydroquinoline wherein a total mass fraction of dimers, trimers, and tetramers of 2,2,4-trimethyl-1,2-dihydroquinoline is ≥ 80%, and a structural formula of the compound of formula A is: 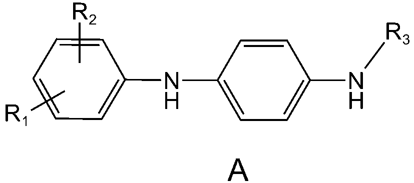 in formula A, R₁, R₂ and R₃ are each independently selected from C1-C8 alkyl;
preferably, in formula A, R₁ and R₂ are each independently selected from C1-C2 alkyl, and R₃ is selected from C3-C6 alkyl, preferably selected from C4-C6 alkyl.

5. The antidegradant composition of claim 4, wherein the compound of formula A is selected from a compound of formula I, a compound of formula II, a compound of formula IV, and a compound of formula VI:

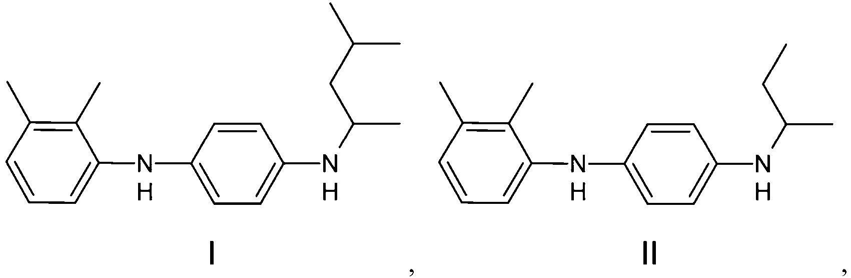

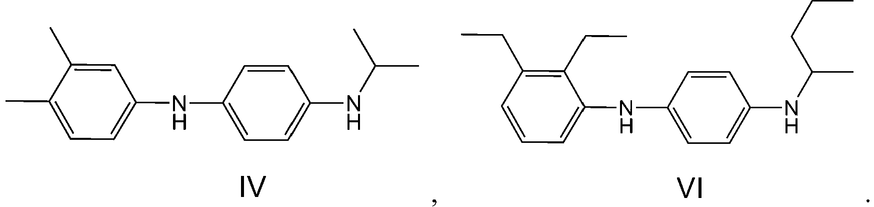

6. The antidegradant composition of claim 4, wherein in the antidegradant composition, a mass ratio of the compound of formula A to the antidegradant STMQ is 1:1 to 3:1, preferably 1.5:1 to 2.5:1.

7. A rubber composition, wherein raw materials of the rubber composition comprise 100 parts by mass of a diene elastomer and 1 to 5 parts by mass of a compound of formula A or the antidegradant composition according to any one of claims 4-6, and a structural formula of the compound of formula A is: 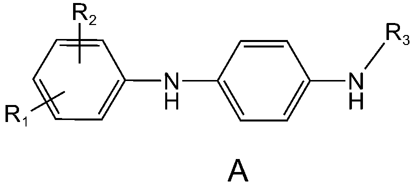 in formula A, R₁, R₂ and R₃ are each independently selected from C1-C8 alkyl;
preferably, in formula A, R₁ and R₂ are each independently selected from C1-C2 alkyl, and R₃ is selected from C3-C6 alkyl, preferably selected from C4-C6 alkyl;
preferably, the compound of formula A is selected from a compound of formula I, a compound of formula II, a compound of formula IV, and a compound of formula VI:

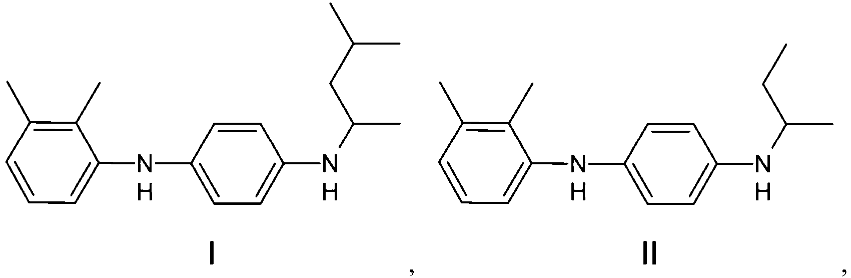

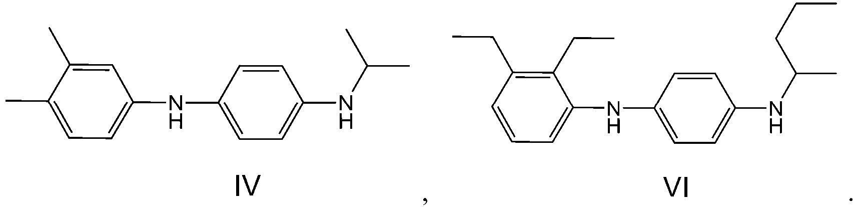

8. The rubber composition of claim 4, wherein the rubber composition has one or more of the following characteristics:
the diene elastomer comprises natural rubber and butadiene rubber; preferably, a mass ratio of the natural rubber to the butadiene rubber is 1:2 to 2:1;
the raw materials of the rubber composition further comprise 30 to 70 parts by mass of a reinforcing filler; preferably, the reinforcing filler is carbon black;
the raw materials of the rubber composition further comprise 1 to 10 parts by mass of an activator; preferably, the activator is zinc oxide;
the raw materials of the rubber composition further comprise 2 to 15 parts by mass of a softener; preferably, the softener comprises aromatic oil and stearic acid in a mass ratio of 1:1 to 5:1;
the raw materials of the rubber composition further comprise 0.5 to 3 parts by mass of sulfur;
the raw materials of the rubber composition further comprise 0.2 to 2 parts by mass of an accelerator, and the accelerator is preferably N-tert-butylbenzothiazole-2-sulphenamide.

9. A rubber product, wherein the rubber product comprises the rubber composition according to claim 7 or 8; preferably, the rubber product is a tire.

10. Use of a compound of formula A or the antidegradant composition according to any one of claims 4-6 in improving damp-heat aging resistance and/or ozone aging resistance of a rubber composition or a rubber product, and a structural formula of the compound of formula A is: 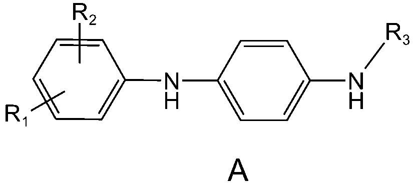 in formula A, R₁, R₂ and R₃ are each independently selected from C1-C8 alkyl;
preferably, in formula A, R₁ and R₂ are each independently selected from C1-C2 alkyl, and R₃ is selected from C3-C6 alkyl, preferably selected from C4-C6 alkyl;
preferably, the compound of formula A is selected from a compound of formula I, a compound of formula II, a compound of formula IV, and a compound of formula VI:
